(19) Europäisches Patentamt

European Patent Office

Office européen des brevets

(11) **EP 0 819 173 B1**

(12) **EUROPÄISCHE PATENTSCHRIFT**

(45) Veröffentlichungstag und Bekanntmachung des Hinweises auf die Patenterteilung:
**06.05.2004 Patentblatt 2004/19**

(51) Int Cl.⁷: **C12N 15/15**, C07K 14/81, C12N 9/64, C12N 9/72, C12N 1/21, C12N 5/10

(21) Anmeldenummer: **96909149.5**

(22) Anmeldetag: **29.03.1996**

(86) Internationale Anmeldenummer:
**PCT/EP1996/001388**

(87) Internationale Veröffentlichungsnummer:
**WO 1996/031606 (10.10.1996 Gazette 1996/45)**

(54) **MUTANT DES INHIBITORS DES ERYTHRINA CAFFRA TYPS UND DESSEN VERWENDUNG ZUR REINIGUNG VON SERINPROTEASEN**

MUTANT OF THE ERYTHRINA CAFFRA TYPE INHIBITOR AND THE USE OF THE SAID MUTANT FOR PURIFYING SERIN PROTEASES

MUTANT DE L'INHIBITEUR DU TYPE ERYTHRINA CAFFRA ET SON UTILISATION POUR LA PURIFICATION DES SERINE-PROTEASES

(84) Benannte Vertragsstaaten:
**AT BE CH DE DK ES FI FR GB GR IE IT LI LU NL PT SE**

(30) Priorität: **06.04.1995 DE 19512937**

(43) Veröffentlichungstag der Anmeldung:
**21.01.1998 Patentblatt 1998/04**

(73) Patentinhaber: **Roche Diagnostics GmbH**
**68305 Mannheim (DE)**

(72) Erfinder:
• **KOHNERT, Ulrich**
**D-82392 Habach (DE)**
• **STERN, Anne**
**D-82377 Penzberg (DE)**
• **WOZNY, Manfred**
**D-82362 Weilheim (DE)**
• **FISCHER, Stephan**
**D-82398 Polling (DE)**

(56) Entgegenhaltungen:
**EP-A- 0 218 479**

• **BIOCHIMICA ET BIOPHISICA ACTA, Bd. 1217, 1994, Seiten 23-28, XP000576154 A. TEIXEIRA ET AL: "Site-directed mutagenesis of the synthetic Erythrina trypsin/tissue plasminogen activator (tPA) inhibitor....." in der Anmeldung erwähnt**

SEQUENZPROTOKOLL

(1) ALLGEMEINE ANGABEN:

    (i) ANMELDER:

        (A) NAME: BOEHRINGER MANNHEIM GMBH
        (B) STRASSE: Sandhofer Str. 116
        (C) ORT: Mannheim
        (E) LAND: Germany
        (F) POSTLEITZAHL: D-68305
        (G) TELEFON: 08856/60-3446
        (H) TELEFAX: 08856/60-3451

    (ii) BEZEICHNUNG DER ERFINDUNG: Neuer Inhibitor des Erythrina caffra Typs und dessen Verwendung zur Reinigung von Serinproteasen

    (iii) ANZAHL DER SEQUENZEN: 10

    (iv) COMPUTER-LESBARE FASSUNG:

        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.30B (EPA)

    (vi) DATEN DER URANMELDUNG:

        (A) ANMELDENUMMER: DE 195 12 937.7
        (B) ANMELDETAG: 06-APR-1995

(2) ANGABEN ZU SEQ ID NO: 1:

    (i) SEQUENZKENNZEICHEN:

        (A) LÄNGE: 516 Basenpaare
        (B) ART: Nucleotid
        (C) STRANGFORM: Doppelstrang

(D) TOPOLOGIE: linear

(ii) ART DES MOLEKÜLS: cDNA

(ix) MERKMAL:

    (A) NAME/SCHLÜSSEL: CDS
    (B) LAGE:1..516

(xi) SEQUENZBESCHREIBUNG: SEQ ID NO: 1:

```
TCA TTA TTA GAT GGT AAC GGC GAA GTG GTG CAG AAC GGC GGT ACC TAT        48
Ser Leu Leu Asp Gly Asn Gly Glu Val Val Gln Asn Gly Gly Thr Tyr
 1           5                   10                  15


TAT CTG CTG CCG CAG GTG TGG GCG CAG GGC GGC GGC GTG CAG CTG GCG        96
Tyr Leu Leu Pro Gln Val Trp Ala Gln Gly Gly Gly Val Gln Leu Ala
            20              25              30

AAA ACC GGC GAA GAA ACC TGC CCG CTG ACC GTG GTG CAG AGC CCG AAC       144
Lys Thr Gly Glu Glu Thr Cys Pro Leu Thr Val Val Gln Ser Pro Asn
            35              40              45

GAA CTG AGC GAT GGC AAA CCG ATT CGT ATT GAA AGC CGT CTG CGT AGC       192
Glu Leu Ser Asp Gly Lys Pro Ile Arg Ile Glu Ser Arg Leu Arg Ser
     50                  55                  60

GCG TTT ATT CCG GAT GAT GAT AAA GTG CGT ATT GGC TTT GCG TAT GCG       240
Ala Phe Ile Pro Asp Asp Asp Lys Val Arg Ile Gly Phe Ala Tyr Ala
 65                  70                  75                  80

CCG AAA TGC GCG CCG AGC CCG TGG TGG ACC GTG GTG GAA GAT GAA CAG       288
Pro Lys Cys Ala Pro Ser Pro Trp Trp Thr Val Val Glu Asp Glu Gln
                85                  90                  95

GAA GGC CTG AGC GTG AAA CTG AGC GAA GAT GAA AGC ACC CAG TTT GAT       336
Glu Gly Leu Ser Val Lys Leu Ser Glu Asp Glu Ser Thr Gln Phe Asp
            100                 105                 110

TAT CCG TTT AAA TTT GAA CAG GTG AGC GAT CAG CTG CAT AGC TAT AAA       384
Tyr Pro Phe Lys Phe Glu Gln Val Ser Asp Gln Leu His Ser Tyr Lys
            115                 120                 125

CTG CTG TAT TGC GAA GGC AAA CAT GAA AAA TGC GCG AGC ATT GGC ATT       432
Leu Leu Tyr Cys Glu Gly Lys His Glu Lys Cys Ala Ser Ile Gly Ile
            130                 135                 140

AAC CGT GAT CAG AAA GGC TAT CGT CGT CTG GTG GTG ACC GAA GAT TAT       480
Asn Arg Asp Gln Lys Gly Tyr Arg Arg Leu Val Val Thr Glu Asp Tyr
145                 150                 155                 160

CCG CTG ACC GTG GTG CTG AAA AAA GAT GAA AGC AGC                       516
Pro Leu Thr Val Val Leu Lys Lys Asp Glu Ser Ser
                165                 170
```

(2) ANGABEN ZU SEQ ID NO: 2:

(i) SEQUENZKENNZEICHEN:

(A) LÄNGE: 172 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 2:

```
Ser Leu Leu Asp Gly Asn Gly Glu Val Val Gln Asn Gly Gly Thr Tyr
 1               5                   10                  15

    Tyr Leu Leu Pro Gln Val Trp Ala Gln Gly Gly Gly Val Gln Leu Ala
                 20                  25                  ᴐ

    Lys Thr Gly Glu Glu Thr Cys Pro Leu Thr Val Val Gln Ser Pro Asn
             35                  40                  45

    Glu Leu Ser Asp Gly Lys Pro Ile Arg Ile Glu Ser Arg Leu Arg Ser
         50                  55                  60

    Ala Phe Ile Pro Asp Asp Asp Lys Val Arg Ile Gly Phe Ala Tyr Ala
     65                  70                  75                  80

    Pro Lys Cys Ala Pro Ser Pro Trp Trp Thr Val Val Glu Asp Glu Gln
                 85                  90                  95

    Glu Gly Leu Ser Val Lys Leu Ser Glu Asp Glu Ser Thr Gln Phe Asp
                100                 105                 110

    Tyr Pro Phe Lys Phe Glu Gln Val Ser Asp Gln Leu His Ser Tyr Lys
             115                 120                 125

    Leu Leu Tyr Cys Glu Gly Lys His Glu Lys Cys Ala Ser Ile Gly Ile
         130                 135                 140

    Asn Arg Asp Gln Lys Gly Tyr Arg Arg Leu Val Val Thr Glu Asp Tyr
    145                 150                 155                 160

    Pro Leu Thr Val Val Leu Lys Lys Asp Glu Ser Ser
                    165                 170
```

(2) ANGABEN ZU SEQ ID NO: 3:

(i) SEQUENZKENNZEICHEN:

(A) LÄNGE: 4 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 3:

```
    Val Leu Leu Asp
    1
```

(2) ANGABEN ZU SEQ ID NO: 4:

(i) SEQUENZKENNZEICHEN:

(A) LÄNGE: 4 Aminosäuren
(B) ART: Aminosäure
(C) STRANGFORM: Einzelstrang
(D) TOPOLOGIE: linear

(ii) ART DES MOLEKÜLS: Peptid

(xi) SEQUENZBESCHREIBUNG: SEQ ID NO: 4:

```
                            Ser Leu Leu Asp
                            1
```

(2) ANGABEN ZU SEQ ID NO: 5:

(i) SEQUENZKENNZEICHEN:

(A) LÄNGE: 27 Basenpaare
(B) ART: Nucleotid
(C) STRANGFORM: Einzelstrang
(D) TOPOLOGIE: linear

(ii) ART DES MOLEKÜLS: other nucleic acid

(A) BESCHREIBUNG: /desc = "Primer"

(xi) SEQUENZBESCHREIBUNG: SEQ ID NO: 5:

```
AAAGGATCCC TCGAGAAATC ATAAAAA                                    27
```

(2) ANGABEN ZU SEQ ID NO: 6:

(i) SEQUENZKENNZEICHEN:

(A) LÄNGE: 34 Basenpaare
(B) ART: Nucleotid
(C) STRANGFORM: Einzelstrang
(D) TOPOLOGIE: linear

(ii) ART DES MOLEKÜLS: other nucleic acid

(A) BESCHREIBUNG: /desc = "Primer"

(xi) SEQUENZBESCHREIBUNG: SEQ ID NO: 6:

CATAAGAATT CTGTTTCCTC TTTAATGAAT TCTG                                     34

(2) ANGABEN ZU SEQ ID NO: 7:

(i) SEQUENZKENNZEICHEN:

(A) LÄNGE: 24 Basenpaare
(B) ART: Nucleotid
(C) STRANGFORM: Einzelstrang
(D) TOPOLOGIE: linear

(ii) ART DES MOLEKÜLS: other nucleic acid

(A) BESCHREIBUNG: /desc = "primer"

(xi) SEQUENZBESCHREIBUNG: SEQ ID NO: 7:

CAGAATTCTT ATGTCATTAT TAGA                                     24

(2) ANGABEN ZU SEQ ID NO: 8:

(i) SEQUENZKENNZEICHEN:

(A) LÄNGE: 18 Basenpaare
(B) ART: Nucleotid
(C) STRANGFORM: Einzelstrang
(D) TOPOLOGIE: linear

(ii) ART DES MOLEKÜLS: other nucleic acid

(A) BESCHREIBUNG: /desc = "Primer"

(xi) SEQUENZBESCHREIBUNG: SEQ ID NO: 8:

AGAAGCTTTT ATCAGCTG                                     18

(2) ANGABEN ZU SEQ ID NO: 9:

(i) SEQUENZKENNZEICHEN:

(A) LÄNGE: 541 Basenpaare
(B) ART: Nucleotid
(C) STRANGFORM: Doppelstrang
(D) TOPOLOGIE: linear

(ii) ART DES MOLEKÜLS: cDNA

(ix) MERKMAL:

(A) NAME/SCHLÜSSEL: CDS

(B) LAGE:11..529

(ix) MERKMAL:

(A) NAME/SCHLÜSSEL: mat_peptide

(B) LAGE:11

(xi) SEQUENZBESCHREIBUNG: SEQ ID NO: 9:

```
CAGAATTCTT ATG TCA TTA TTA GAT GGT AAC GGC GAA GTG GTG CAG AAC        49
           Met Ser Leu Leu Asp Gly Asn Gly Glu Val Val Gln Asn
            1               5                   10

GGC GGT ACC TAT TAT CTG CTG CCG CAG GTG TGG GCG CAG GGC GGC GGC        97
Gly Gly Thr Tyr Tyr Leu Leu Pro Gln Val Trp Ala Gln Gly Gly Gly
     15              20                  25

GTG CAG CTG GCG AAA ACC GGC GAA GAA ACC TGC CCG CTG ACC GTG GTG       145
Val Gln Leu Ala Lys Thr Gly Glu Glu Thr Cys Pro Leu Thr Val Val
 30              35                  40                      45

CAG AGC CCG AAC GAA CTG AGC GAT GGC AAA CCG ATT CGT ATT GAA AGC       193
Gln Ser Pro Asn Glu Leu Ser Asp Gly Lys Pro Ile Arg Ile Glu Ser
                 50                  55                  60

CGT CTG CGT AGC GCG TTT ATT CCG GAT GAT GAT AAA GTG CGT ATT GGC       241
Arg Leu Arg Ser Ala Phe Ile Pro Asp Asp Asp Lys Val Arg Ile Gly
                 65                  70                  75

TTT GCG TAT GCG CCG AAA TGC GCG CCG AGC CCG TGG TGG ACC GTG GTG       289
Phe Ala Tyr Ala Pro Lys Cys Ala Pro Ser Pro Trp Trp Thr Val Val
             80                  85                  90

GAA GAT GAA CAG GAA GGC CTG AGC GTG AAA CTG AGC GAA GAT GAA AGC       337
Glu Asp Glu Gln Glu Gly Leu Ser Val Lys Leu Ser Glu Asp Glu Ser
         95                  100                 105

ACC CAG TTT GAT TAT CCG TTT AAA TTT GAA CAG GTG AGC GAT CAG CTG       385
Thr Gln Phe Asp Tyr Pro Phe Lys Phe Glu Gln Val Ser Asp Gln Leu
110                 115                 120                     125

CAT AGC TAT AAA CTG CTG TAT TGC GAA GGC AAA CAT GAA AAA TGC GCG       433
His Ser Tyr Lys Leu Leu Tyr Cys Glu Gly Lys His Glu Lys Cys Ala
                 130                 135                 140

AGC ATT GGC ATT AAC CGT GAT CAG AAA GGC TAT CGT CGT CTG GTG GTG       481
Ser Ile Gly Ile Asn Arg Asp Gln Lys Gly Tyr Arg Arg Leu Val Val
             145                 150                 155

ACC GAA GAT TAT CCG CTG ACC GTG GTG CTG AAA AAA GAT GAA AGC AGC       529
Thr Glu Asp Tyr Pro Leu Thr Val Val Leu Lys Lys Asp Glu Ser Ser
             160                 165                 170

TGATAAAAGC TT                                                         541
```

(2) ANGABEN ZU SEQ ID NO: 10:

(i) SEQUENZKENNZEICHEN:

    (A) LÄNGE: 173 Aminosäuren
    (B) ART: Aminosäure
    (D) TOPOLOGIE: linear

(ii) ART DES MOLEKÜLS: Protein

(xi) SEQUENZBESCHREIBUNG: SEQ ID NO: 10:

```
Met Ser Leu Leu Asp Gly Asn Gly Glu Val Val Gln Asn Gly Gly Thr
 1               5                  10                      15

Tyr Tyr Leu Leu Pro Gln Val Trp Ala Gln Gly Gly Gly Val Gln Leu
            20                  25                  30

Ala Lys Thr Gly Glu Glu Thr Cys Pro Leu Thr Val Val Gln Ser Pro
        35                  40                  45

Asn Glu Leu Ser Asp Gly Lys Pro Ile Arg Ile Glu Ser Arg Leu Arg
        50                  55                  60

Ser Ala Phe Ile Pro Asp Asp Asp Lys Val Arg Ile Gly Phe Ala Tyr
65                  70                  75                      80

Ala Pro Lys Cys Ala Pro Ser Pro Trp Trp Thr Val Val Glu Asp Glu
                85                  90                  95

Gln Glu Gly Leu Ser Val Lys Leu Ser Glu Asp Glu Ser Thr Gln Phe
            100                 105                 110

Asp Tyr Pro Phe Lys Phe Glu Gln Val Ser Asp Gln Leu His Ser Tyr
        115                 120                 125

Lys Leu Leu Tyr Cys Glu Gly Lys His Glu Lys Cys Ala Ser Ile Gly
        130                 135                 140

Ile Asn Arg Asp Gln Lys Gly Tyr Arg Arg Leu Val Val Thr Glu Asp
145                 150                 155                     160

Tyr Pro Leu Thr Val Val Leu Lys Lys Asp Glu Ser Ser
                165                 170
```

## Beschreibung

Die Erfindung betrifft einen neuen Inhibitor des Erythrina caffra Typs und dessen Verwendung zur Reinigung von Serinproteasen.

Immobilisierte Trypsin-Inhibitoren aus Erythrina (ETI) sind effektive Reagenzien für die affinitätschromatographische Reinigung von Serinproteasen, insbesondere von Plasminogenaktivatoren (C. Heussen (1984) *(22)*, β-Trypsin, α-Chymotrypsin und Thrombin (S. Onesti et al. (1992) *(34)*. Diese Trypsin-Inhibitoren sind seit langem bekannt (C. Heussen (1982) *(23)*; F.J. Joubert (1982) *(26)*; F.J. Joubert (1982) *(27)*.

Der Inhibitor DE-3 aus E. caffra ist besonders bevorzugt zur Reinigung von Plasminogenaktivatoren geeignet (F.J. Joubert (1987) *(25)*. In dieser Publikation ist auch die komplette Aminosäuresequenz dieses Inhibitors beschrieben. DE-3 kann aus dem Samen von E. caffra isoliert und gereinigt werden (F.J. Joubert, (1982) *(26)*). Ein ETI, welcher nicht cytotoxisch ist, ist in der EP-B 0 218 479 *(15)* beschrieben.

Von Teixeira et al. (1994) *(45)* wird ein rekombinanter ETI beschrieben, dessen spezifische inhibitorische Aktivität für Gewebsplasminogenaktivator bei $1,7 \times 10^9$ U/mmol liegt. Die spezifische inhibitorische Aktivität von natürlichem ETI liegt dagegen bei $1,94 \times 10^9$ U/mmol. Analoges gilt für die inhibitorische Aktivität gegenüber Trypsin ($2,63 \times 10^{12}/3,21 \times 10^{12}$). Damit ist die spezifische inhibitorische Aktivität von nach Teixeira hergestelltem rekombinantem ETI für Trypsin um 20% und für Gewebsplasminogenaktivator um 10% geringer als die Aktivität von natürlichem ETI.

Von Teixeira et al. (1994) *(46)* wird eine modifizierte Form von ETI beschrieben, in welcher das N-terminale Val gegen Asp ausgetauscht ist. Ein so modifizierter ETI bindet nicht an tPA und zeigt auch keine inhibitorische Aktivität gegenüber tPA. Die spezifische inhibitorische Aktivität gegenüber Trypsin ist für natürlichen ETI und Asp-modifizierten ETI praktisch identisch.

Rekombinanter ETI wird nach Teixeira durch Expression gewonnen und aufgereinigt durch eine Ammoniumsulfatfällung (80%ige Sättigung), Dialyse gegen Wasser und eine Cyanbromidspaltung, wobei die N-terminale Sequenz einschließlich des Methionins abgespalten wird. Anschließend wird über Gelfiltration (Sephadex G50) gereinigt.

In der DE-A 44 24 171.2 *(9)* ist ein gereinigtes Polypeptid, welches die Aktivität eines Inhibitiors DE-3 aus Erythrina caffra besitzt (im weiteren auch als ETI-Polypeptid bezeichnet), beschrieben, welches im Gegensatz zum aus natürlichen Quellen isolierten Inhibitor eine deutlich erhöhte Spezifität gegenüber Serinproteasen besitzt.

Ein weiteres wesentliches Kriterium für die Eignung eines ETI-Polypeptids zur effektiven Reinigung von Serinproteasen ist die Bindungskapazität für Serinproteasen.

Aufgabe der Erfindung ist demnach die Verbesserung der Effektivität der Bindungskapazität eines ETI-Polypeptids zur Reinigung von Serinproteasen.

Gegenstand der Erfindung ist ein Polypeptid, welches die Aktivität eines Inhibitors DE-3 aus Erythrina caffra besitzt, reversibel eine Serinprotease aus einem Proteingemisch selektiv bindet und wobei das Polypeptid eine Aminosäuresequenz besitzt, welche funktionell analog zur SEQ ID NO:2 ist, in einem Teilbereich zu mehr als 85 % homolog zum Bereich der Aminosäuren 39 - 139 dieser Sequenz ist, zwei Disulfidbrücken besitzt und N-terminal mit Ser, vorzugsweise mit SEQ ID NO:4 beginnt. Die Bindungskapazität dieses Polypeptids für Plasminogenaktivatoren liegt bei 1,25 MU/ml und mehr, und das Polypeptid besitzt eine spezifische Aktivität von 1,07 U/mg oder mehr für Trypsin.

Vorzugsweise wird ein erfindungsgemäßes Polypeptid codiert

a) von einer Nukleinsäure gemäß SEQ ID NO:1,

b) von einer Nukleinsäure, die mit der in SEQ ID NO:1 gezeigten DNA-Sequenz unter stringenten Bedingungen hybridisiert und N-terminal mit einer Nukleinsäuresequenz beginnt, welche für SEQ ID NO:4 codiert,

c) von einer Nukleinsäure, die ohne die Degeneration des genetischen Codes mit einer der in a) oder b) genannten Sequenzen unter stringenten Bedingungen hybridisieren würde.

Es hat sich überraschenderweise gezeigt, daß durch den Austausch des N-terminalen Val gegen Ser in einem ETI-Polypeptid die Bindungskapazität für Serinproteasen deutlich erhöht wird. Während das bekannte ETI-Polypeptid N-terminal mit der Aminosäuresequenz Val-Leu-Leu-Asp (SEQ-ID-NO:3) beginnt, beginnt das ETI-Polypeptid gemäß der Erfindung mit Ser-Leu-Leu-Asp (SEQ-ID-NO:4).

Diese Ergebnisse sind besonders überraschend im Hinblick auf Teixeira et al. (1994) *(46)*. Aus dieser Publikation mußte ein Fachmann erwarten, daß die Modifikation des N-Terminus von ETI dazu führt, daß ein so modifizierter ETI seine spezifische inhibitorische Aktivität gegenüber Plasminogenaktivatoren verliert und seine Aktivität gegenüber Trypsin sich nicht verändern wird. Um so überraschender ist, daß für einen erfindungsgemäß modifizierten ETI sowohl die spezifische Aktivität für Trypsin als auch die Bindekapazität für Plasminogenaktivatoren, insbesondere Gewebsplasminogenaktivatoren, in beträchtlichem Umfang zunimmt.

Unter "einem Polypeptid mit der Aktivität eines Inhibitors DE-3 aus Erythrina caffra" ist ein Polypeptid zu verstehen, welches spezifisch Serinproteasen, wie Plasminogenaktivatoren, β-Trypsin, α-Chymotrypsin und/oder Thrombin spezifisch bindet. Durch die Bindung kann die Aktivität der Serinprotease inhibiert werden.

Unter einem "funktionell analogen" ETI-Polypeptid ist ein Polypeptid zu verstehen, welches die Aktivität eines Inhibitors DE-3 aus Erythrina caffra besitzt. Modifikationen der Proteinsequenz sind in dem dem Fachmann geläufigen üblichen Rahmen möglich. Dabei ist jedoch zu beachten, daß der N-Terminus identisch mit SEQ ID NO:4 sein sollte (N-terminales Ser) und das Polypeptid zwei Disulfidbrücken, zur Fixierung der Raumstruktur, besitzen muß. Nach rekombinanter Herstellung in Prokaryonten kann das erfindungsgemäße Protein auch noch ein N-terminales Methionin enthalten (SEQ ID NO:10). Dabei sollen diese Disulfidbrücken in ihrer Lage den Disulfidbrücken des ETI-Polypeptids (Cys 39 - Cys 83 und Cys 132 - Cys 139, Lehle, K. et al. (1994) *(30)*) entsprechen. Ebenso sollte das ETI-Polypeptid in einem Teilbereich zu mehr als 85 % homolog zu dem Sequenzbereich 39 - 139 von SEQ ID NO:2 sein. Dieser Teilbereich ist vorzugsweise ebenfalls der Sequenzbereich 39 - 139 des erfindungsgemäßen Proteins und vorzugsweise identisch oder im wesentlichen identisch mit dem Sequenzbereich 39 - 139 aus SEQ ID NO:2.

Besonders bevorzugt wird ein ETI-Polypeptid (oder eine Nukleinsäure, die für ein solches Polypeptid codiert) verwendet, dessen Aminosäuresequenz identisch oder im wesentlichen identisch mit SEQ ID NO:2 ist. Es hat sich überraschenderweise weiter gezeigt, daß die Bindekapazität des Inhibitors für Serinproteasen dann besonders hoch ist, wenn nach rekombinanter Herstellung in Prokaryonten das N-terminale Methionin vollständig oder zumindest weitgehend (vorzugsweise zu mehr als 85 % in der ETI-Polypeptidpräparation) abgespalten ist. Das ETI-Polypeptid kann unterschiedlich groß sein. Vorzugsweise umfaßt es jedoch 100 - 200 Aminosäuren, besonders bevorzugt etwa 139 - 173 Aminosäuren.

Unter der Bindungskapazität des Inhibitors für Serinproteasen ist die Menge einer Serinprotease (vorzugsweise ein Plasminogenaktivator) zu verstehen, welche sich durch einen immobilisierten Inhibitor aus einer Lösung entfernen läßt. Die Bindungskapazität wird üblicherweise als Aktivität/ml Matrix [bei Plasminogenaktivatoren als amidolytische Aktivität in U/ml Matrix] angegeben. Die Aktivität des Plasminogenaktivators wird zweckmäßig nach Elution der zuvor gebundenen Serinprotease bestimmt.

Die Bestimmung der Aktivität erfolgt nach U. Kohnert et al. (1992) *(29)*. Dazu wird die Geschwindigkeit der Spaltung von H-D-Ile-Pro-Arg-p-Nitroanilid-Dihydrochlorid (S2288, Kabi Vitrum, Schweden) über die Absorption bei 405 nm photometrisch gemessen.

Ein weiterer Gegenstand der Erfindung ist ein Verfahren zur Reinigung einer Serinprotease aus einem Proteingemisch durch Bindung der Serinprotease an ein immobilisiertes ETI-Polypeptid, welches reversibel Serinproteasen selektiv bindet,. entfernen der ungebundenen Anteile aus dem Proteingemisch, Ablösen der Serinprotease vom Inhibitor, Trennung des immobilisierten Inhibitors von der löslichen Serinprotease und Isolierung der Serinprotease, welches dadurch gekennzeichnet ist, daß ein erfindungsgemäßes ETI-Polypeptid verwendet wird, welches reversibel Serinproteasen aus einem Proteingemisch selektiv bindet und wobei das Polypeptid vorzugsweise eine Aminosäuresequenz besitzt, welche funktionell analog zur SEQ ID NO:2 ist, in einem Teilbereich zu mehr als 85 % homolog zum Bereich der Aminosäuren 39 - 139 dieser Sequenz ist, zwei Disulfidbrücken besitzt, N-terminal mit SEQ ID NO:4 oder mit einer um Methionin N-terminal verlängerten SEQ ID NO:4 beginnt und eine spezifische Aktivität von 1,07 U/mg oder höher gegen Trypsin und eine Bindekapazität von 1,25 MU/ml oder mehr für Plasminogenaktivatoren besitzt.

Vorzugsweise wird ein solches ETI-Polypeptid durch prokaryontische oder eukaryontische Expression einer exogenen DNA hergestellt. Die Reinigung und Isolierung erfolgt vorzugsweise chromatographisch über einen Anionentauscher, Kationentauscher oder eine Nickelchelatsäule.

Das erfindungsgemäße Verfahren ist besonders vorteilhaft zur Reinigung von Plasminogenaktivatoren, wie Gewebs-Plasminogen-Aktivatoren (t-PA) und Derivate (z.B. Mutationen und Deletionen) davon, geeignet. t-PA ist in der EP-B 0 093 619 *(13)*, Derivate von t-PA sind in US-Patent 5,223,256 *(53)*, WO 90/09437 *(54)* und T.J.R. Harris (1987) *(20)* beschrieben.

Die Herstellung des ETI-Polypeptids kann nach den dem Fachmann geläufigen Methoden erfolgen. Dazu wird zunächst ein Nukleinsäuremolekül (vorzugsweise DNA) hergestellt, welches für ein ETI-Polypeptid codiert, welches N-terminal mit SEQ ID NO:4 oder mit einer um Methionin N-terminal verlängerten SEQ ID NO:4 beginnt und für ein ETI-Polypeptid codiert. Das ETI-Polypeptid besitzt eine Aminosäuresequenz, die funktionell analog zu SEQ ID NO: 2 ist, und enthält einen Teilbereich, der zu mehr als 85 %, vorzugsweise vollständig homolog zum Bereich der Aminosäuren 39 -139 von SEQ ID NO:2 ist, und zwei Disulfidbrücken. Dabei kann auch eine Sequenz verwendet werden, die im Rahmen der Degeneration des genetischen Codes für das gleiche Polypeptid codiert und/oder komplementär zu dieser Sequenz ist. Vorzugsweise wird auch eine Nukleinsäure verwendet, die mit SEQ ID NO:1 unter stringenten Bedingungen hybridisiert und N-terminal für SEQ ID NO:4 oder für eine um Methionin N-terminal verlängerte SEQ ID NO:4 codiert. Die DNA wird in einen Vektor kloniert, der in eine Wirtszelle transferiert werden kann und dort replizierbar ist. Ein derartiger Vektor enthält zusätzlich zur ETI-Polypeptidsequenz Operator-Elemente, die zur Expression der DNA erforderlich sind. Dieser Vektor, der die Inhibitor-DNA und die Operator-Elemente enthält, wird in einen Vektor transferiert, der in der Lage ist, die DNA des ETI-Polypeptids zu exprimieren. Die Wirtszelle wird unter Bedingungen kultiviert, welche die Expression des ETI-Polypeptids erlauben. Das ETI-Polypeptid wird aus diesen Zellen gewonnen. Dabei wird durch geeignete Maßnahmen sichergestellt, daß das ETI-Polypeptid eine aktive tertiäre Struktur einnehmen kann, in der es Inhibitoreigenschaften zeigt.

Dabei ist es, wie bereits ausgeführt, nicht erforderlich, daß das ETI-Polypeptid die exakte Aminosäuresequenz entsprechend SEQ ID NO:2 und SEQ ID NO:4 besitzt. Ebenso geeignet sind ETI-Polypeptide, welche im wesentlichen die gleiche Sequenz besitzen und Polypeptide mit der Aktivität eines Inhibitors DE-3 aus Erythrina caffra sind. Wesentlich ist jedoch, daß am N-Terminus Val gegen Ser ausgetauscht ist. Die Aminosäuresequenzen SEQ ID NO:2 und 4 werden bevorzugt verwendet, wobei im Falle der Expression in prokaryontischen Wirtszellen, nicht jedoch nach eukaryontischer Expression, ein N-terminales Methionin enthalten sein kann (SEQ ID NO:10). Üblicherweise wird jedoch auch in E.coli das Methionin abgespalten, da die Sequenz N-terminal mit Met-Ser beginnt (Dalborge H. et al. (1990) (7)). Ein solches Polypeptid, in dem das Met abgespalten ist, ist bevorzugt.

Ein weiterer Gegenstand der Erfindung ist eine isolierte Nukleinsäure, welche für ein ETI-Polypeptid codiert, welches reversibel Serinproteasen aus einem Proteingemisch selektiv bindet und wobei das Protein eine Aminosäuresequenz besitzt, welche funktionell analog zur SEQ ID NO:2 ist, in einem Teilbereich zu mehr als 85 % homolog zum Bereich der Aminosäuren 39 - 139 dieser Sequenz ist, zwei Disulfidbrücken besitzt und N-terminal mit SEQ ID NO:4 oder mit einer um Methionin N-terminal verlängerten SEQ ID NO:4 beginnt. Eine solche isolierte Nukleinsäure ist vorzugsweise identisch mit SEQ ID NO:1 oder mit einer Nukleinsäure, die im Rahmen der Degeneration des genetischen Codes für das gleiche Polypeptid codiert. Zur Expression in eukaryontischen oder prokaryontischen Wirtszellen enthält die Nukleinsäure am 5'-Ende die dem Fachmann geläufigen eukaryontischen oder prokaryontischen Transkriptions- und Translationssignale.

Vorzugsweise wird eine Nukleinsäure verwendet, welche unter stringenten Bedingungen mit SEQ ID NO:1 unter Standardbedingungen hybridisiert. Solche Standardbedingungen und Verfahren zur Hybridisierung sind dem Fachmann bekannt und beispielsweise von J. Sambrook et al. (1989) (38) und B.D. Hames, S.G. Higgins (1985) (19) beschrieben. Die dort beschriebenen Standardprotokolle werden üblicherweise hierfür verwendet. Insbesondere wird hingewiesen auf Sambrook, Section IX (40), wobei beide Publikationen Gegenstand der Offenbarung dieser Erfindung sind. Standardisierte stringente Bedingungen sind ebenso beschrieben in Höltke und Kessler (1990) (24).

Bevorzugte stringente Bedingungen sind gegeben bei einer Hybridisierung in Gegenwart von 1 mol/l NaCl, 1 % SDS und 10 % Dextransulfat bei anschließendem zweifachen Waschen des Filters bei Raumtemperatur für 5 Minuten in 2 x SSC und einem weiteren Waschschritt für 30 Minuten. Dieser weitere Waschschritt kann bei 0,5 x SSC, 0,1 % SDS, vorzugsweise bei 0,2 x SSC, 0,1 % SDS und besonders bevorzugt bei 0,1 x SSC , 0,1 % SDS bei 65°C durchgeführt werden.

Zur Erleichterung der Herstellung der Vektoren oder Optimierung der Expression können Modifikationen angebracht sein. Derartige Modifikationen sind beispielsweise:

- Veränderung der Nukleinsäure, um verschiedene Erkennungssequenzen von Restriktionsenzymen zur Erleichterung der Schritte der Ligation, Klonierung und Mutagenese einzuführen
- Veränderung der Nukleinsäure zum Einbau von bevorzugten Codons für die Wirtszelle
- Ergänzung der Nukleinsäure um zusätzliche Operator-Elemente, um die Expression in der Wirtszelle zu optimieren.

Die Expression des Inhibitors erfolgt vorzugsweise in Mikroorganismen, wie E. coli. Eine Expression in eukaryontischen Zellen, wie Hefe, CHO-Zellen oder Insektenzellen, ist jedoch auch möglich.

Hierzu werden biologisch funktionelle Plasmide oder virale DNA-Vektoren verwendet, welche die erfindungsgemäße Nukleinsäure enthalten. Mit derartigen Vektoren werden prokaryontische oder eukaryontische Wirtszellen stabil transformiert oder transfiziert.

Die Expressionsvektoren müssen einen Promotor enthalten, der die Expression des Inhibitorproteins im Wirtsorganismus erlaubt. Derartige Promotoren sind dem Fachmann bekannt und sind beispielsweise lac Promotor (Chang et al. (1977) (26)), trp (Goeddel et al. (1980) (18)), λPL Promotor (Shimatake et al., (1981) (41)) und T5 Promotor (US-Patent 4,689,406 (49)). Ebenfalls geeignet sind synthetische Promotoren wie beispielsweise tac Promotor (US-Patent 4,551,433 (48)). Ebenso geeignet sind gekoppelte Promotorsysteme wie beispielsweise T7-RNA-Polymerase/ Promotorsystem (Studier et al. (1986) (44)). Ebenso geeignet sind hybride Promotoren aus einem Bacteriophagen-Promotor und der Operator-Region des Mikroorganismus (EP-A 0 267 851 (11)). Zusätzlich zum Promotor ist eine effektive Ribosomenbindungsstelle erforderlich. Für E. coli wird diese Ribosomenbindungsstelle als Shine-Dalgarno (SD)-Sequenz bezeichnet (Shine et al. (1975) (42); J. Sambrook et al. (1989) (39)).

Zur Verbesserung der Expression ist es möglich, das Inhibitorprotein als Fusionsprotein zu exprimieren. In diesem Fall wird üblicherweise eine DNA-Sequenz, welche für den N-terminalen Teil eines endogenen bakteriellen Proteins oder ein anderes stabiles Protein codiert, an das 5'-Ende der für das Inhibitorprotein codierenden Sequenz fusioniert. Beispiele hierfür sind lacZ, trpE.

Nach Expression werden die Fusionsproteine vorzugsweise mit Enzymen (z.B. Faktor Xa) gespalten (Nagai et al. (1984) *(32)*). Weitere Beispiele für die Spaltstellen sind die IgA-Protease-Spaltstelle (WO 91/11520 *(55)*) und die Ubiquitin-Spaltstelle (Miller et al., (1989) *(31)*). In solchen Fällen kann das erfindungsgemäße ETI-Polypeptid noch N-terminal eine oder mehrere zusätzliche Aminosäuren enthalten. Bevorzugt wird jedoch ein ETI-Polypeptid verwendet, das keine zusätzlichen N-terminalen Aminosäuren enthält und N-terminal mit SEQ ID NO:4 beginnt.

Das zunächst als inaktive Inclusion Bodies erhaltene rekombinante Protein kann nach dem Fachmann geläufigen Verfahren in lösliches aktives Protein überführt werden. Dazu werden die Inclusion Bodies beispielsweise mit Guanidinhydrochlorid oder Harnstoff in Gegenwart eines Reduktionsmittels solubilisiert, reduziert, das Reduktionsmittel z.B. durch Dialyse entfernt und das solubilisierte Protein vorzugsweise unter Verwendung eines Redoxsystems, wie reduziertes und oxidiertes Glutathion oder über gemischte Disulfide renaturiert.

Derartige Methoden sind beispielsweise in US-Patent 4,933,434 *(52)*, EP-B 0 241 022 *(16)* und EP-A 0 219 874 *(10)* beschrieben.

Es ist ebenso möglich, die Proteine als aktive Proteine aus den Mikroorganismen zu sekretieren. Hierzu wird vorzugsweise ein Fusionsprotein verwendet, welches aus der Signalsequenz, die für die Sekretion von Proteinen in den verwendeten Wirtsorganismen geeignet ist (US-Patent 4,336,336 *(47)*), und der Nukleinsäure, welche für das Inhibitorprotein codiert, besteht. Das Protein wird dabei entweder in das Medium (bei grampositiven Bakterien) oder in den periplasmatischen Raum (bei gramnegativen Bakterien) sekretiert. Zwischen der Signalsequenz und der für den Inhibitor codierenden Sequenz ist zweckmäßig eine Spaltstelle angebracht, die entweder bei der Prozessierung oder in einem zusätzlichen Schritt die Abspaltung des Inhibitorproteins erlaubt. Derartige Signalsequenzen sind beispielsweise ompA (Ghrayeb et al., (1984) *(17)*), und phoA (Oka et al. (1985) *(33)*.

Zusätzlich enthalten die Vektoren noch Terminatoren. Terminatoren sind DNA-Sequenzen, die das Ende eines Transkriptionsvorganges signalisieren. Sie zeichnen sich meist durch zwei strukturelle Eigenarten aus: eine umgekehrt repetitive G/C-reiche Region, die intramolekular eine Doppelhelix bilden kann, sowie eine Anzahl von U(bzw. T)-Resten. Beispiele sind trp-Attenuator und Terminator in der DNA des Phagen fd sowie rrnB (Brosius et al. (1981) *(5)*).

Zusätzlich enthalten die Expressionsvektoren üblicherweise einen selektierbaren Marker, um transformierte Zellen zu selektieren. Derartige selektierbare Marker sind beispielsweise die Resistenzgene für Ampicillin, Chloramphenicol, Erythromycin, Kanamycin, Neomycin und Tetracyclin (Davies et al. (1978) *(8)*). Ebenso geeignete selektierbare Marker sind die Gene für essentielle Substanzen der Biosynthese von für die Zelle notwendigen Stoffen wie z.B. Histidin, Tryptophan und Leucin.

Es sind eine Vielzahl von geeigneten bakteriellen Vektoren bekannt. Beispielsweise sind für die folgenden Bakterien Vektoren beschrieben: Bacillus subtilis (Palva et al. (1982) *(35)*), E.coli (Aman et al. (1985) *(1)*; Studier et al., (1986) *(44)*), Streptococcus cremoris (Powell et al. (1988) *(37)*), Streptococcus lividans und Streptomyces lividans (US-Patent 4,747,056 *(50)*).

Eine Expression des Inhibitorproteins ist außer in prokaryontischen Mikroorganismen auch in Eukaryonten (wie beispielsweise CHO-Zellen, Hefe oder Insektenzellen) möglich. Als eukaryontisches Expressionssystem werden Hefe- und Insektenzellen bevorzugt. Die Expression in Hefe kann über drei Arten von Hefevektoren (integrierende YI$_P$ (yeast integrating plasmids)-Vektoren, replizierende YRp (yeast replicon plasmids)-Vektoren und episomale YE$_P$ (yeast episomal plasmids)-Vektoren erfolgen. Näheres hierzu ist beispielsweise in S.M. Kingsman et al. (1987) *(28)* beschrieben.

Weitere gentechnologische Verfahren zur Herstellung und Expression von geeigneten Vektoren sind in J. Sambrook et al. (1989) *(39)* beschrieben.

Nach Herstellung erfolgt die Reinigung von rekombinantem ETI chromatographisch über einen Anionenaustauscher, z.B. eine Q-Sepharose®-Säule, einen Kationenaustauscher (z.B. auf Sulfopropylbasis) oder über eine Nikkelchelatsäule wie beispielsweise in Porath, J. & Olin, B. (1983) *(36)* beschrieben. Überraschenderweise wird nach diesem Reinigungsverfahren ein rekombinantes ETI-Polypeptid erhalten, der immobilisiert an BrCN-Sepharose eine erhöhte Bindungskapazität und eine erhöhte spezifische inhibitorische Aktivität für t-PA und tPA-Derivate besitzt.

Ein so hergestelltes und gereinigtes ETI-Polypeptid ist erhältlich durch Kultivieren von prokaryontischen oder eukaryontischen Wirtszellen, die mit einer exogenen DNA-Sequenz, welche für ein ETI-Polypeptid codiert, reversibel Serinproteasen aus einem Proteingemisch selektiv bindet und wobei das Protein eine Aminosäuresequenz besitzt, welche funktionell analog zur SEQ ID NO:2 ist, in einem Teilbereich zu mehr als 85 % homolog zu dem Bereich der Aminosäuren 39 - 139 dieser Sequenz ist, zwei Disulfidbrücken besitzt und N-terminal mit SEQ ID NO:4 oder mit einer um Methionin N-terminal verlängerten SEQ ID NO:4 beginnt, welche in einer Weise transformiert oder transfiziert sind, die es den Wirtszellen erlaubt, unter geeigneten Nährstoffbedingungen das Polypeptid zu exprimieren, und Isolierung des gewünschten Polypeptids, welches gegenüber natürlichem ETI-Polypeptid aus Erythrina caffra eine für humanen tissue plasminogen activator eine höhere Bindungskapazität besitzt. Die Bindungskapazität liegt vorzugsweise zwischen 1,25 und 1,6 MU/ml.

Ein solches Inhibitorderivat besitzt zusätzlich vorzugsweise eine spezifische inhibitorische Aktivität von 1,07 U/mg, vorzugsweise von 1,5 U/mg oder höher gegen Trypsin. Diese hohe Aktivität wird nach chromatographischer Reinigung an einem Anionenaustauscher, Kationenaustauscher oder an einer Nickelchelat-Säule erhalten.

Ein weiterer Gegenstand der Erfindung ist ein Verfahren zur Herstellung eines rekombinanten ETI-Polypeptids durch Kultivieren von prokaryontischen oder eukaryontischen Wirtszellen, die mit einer exogenen DNA-Sequenz, welche für ein ETI-Polypeptid codiert, welches reversibel Serinproteasen aus einem Proteingemisch selektiv bindet und wobei das Protein eine Aminosäuresequenz besitzt, welche funktionell analog zur SEQ ID NO:2 ist, in einem Teilbereich zu mehr als 85 % homolog zu dem Bereich der Aminosäuren 39 - 139 dieser Sequenz ist, zwei Disulfidbrücken besitzt und N-terminal mit SEQ ID NO:4 beginnt und eine spezifische Aktivität von 1,07 U/mg oder höher gegen Trypsin und eine Bindekapazität von 1,25 MU/ml oder mehr für Plasminogenaktivatoren besitzt, in einer Weise transformiert oder transfiziert sind, die es den Wirtszellen erlaubt, unter geeigneten Nährstoffbedingungen das ETI-Polypeptid zu exprimieren, Isolierung des ETI-Polypeptids aus den Wirtszellen, chromatographische Reinigung an einem Anionentauscher, Kationenaustauscher oder an einer Nickelchelatsäule.

Die Reinigung von Serinproteasen unter Verwendung von rekombinantem ETI-Polypeptid erfolgt nach den dem Fachmann geläufigen Verfahren (vgl. z.B. F.J. Joubert (1987) *(25)*). Hierzu wird das ETI-Polypeptid kovalent an eine Matrix (z.B. CNBr-Sepharose-Säule) gebunden und das Proteingemisch, welches die Serinprotease enthält, bei neutralen oder schwach alkalischen Bedingungen auf die Säule gegeben und eine Chromatographie durchgeführt. Die Elution erfolgt durch pH-Absenkung ≤ pH 5,5 oder durch Verwendung von Pufferlösungen, welche chaotrope Agenzien, wie z.B. KSCN, enthalten. Das Eluat hat eine Proteinreinheit von über 95% bezogen auf die Serinprotease.

Die Immobilisierung des Inhibitors und alle weiteren Verfahrensschritte zur Reinigung von Serinprotease und t-PA können in analoger Weise wie für den aus E. caffra isolierten Inhibitor DE-3 durchgeführt werden. Derartige Verfahren sind beispielsweise beschrieben in der EP-B 0 218 479 *(15)*, EP-B 0 112 122 *(14)*, US-Patent 4,902,623 *(51)*. Die Immobilisierung erfolgt zweckmäßig an einen inerten Träger, vorzugsweise an CNBr-Sepharose®.

Die in der Anmeldung genannten Mikroorganismen DSM 3689 und DSM 5443 wurden am 09.04.1986 (DSM 3689) bzw. 13.07.1989 (DMS 5443) bei der Deutschen Sammlung von Mikroorganismen und Zellkulturen GMbH, Mascheroder Weg 1B, D-38124 Braunschweig, hinterlegt.

Die nachfolgenden Beispiele, Publikationen und das Sequenzprotokoll beschreiben die Erfindung, deren Schutzumfang sich aus den Patentansprüchen ergibt, weiter. Die beschriebenen Verfahren sind als Beispiele zu verstehen, die auch noch nach Modifikation den Gegenstand der Erfindung beschreiben.

**Beispiel 1**

**Expression von ETI-Polypeptid in E. coli**

**a) Gensynthese**

Unter Benutzung der von E. coli bevorzugten Codons wurde aus der Aminosäuresequenz des ETI-Polypeptids aus Erythrina caffra (Joubert, F.J. (1987) *(25)*) eine entsprechende Nukleinsäuresequenz abgeleitet und nach der Methode von Beattie und Fowler (1991) *(2)* synthetisch dargestellt. Um die Klonierung zu erleichtern, wurde an das 5'-Ende eine Schnittstelle für das Restriktionsenzym EcoRI und an das 3'-Ende eine Schnittstelle für das Restriktionsenzym HindIII eingefügt. Die synthetisierte Nukleinsäure wurde mit den Enzymen EcoRI und HindIII restringiert und mit dem Klonierungsvektor pBS+ (Stratagene, US, Catalogue No. 211201 *(43)*, Derivat des Phagen fl und Stratagene's pBS Plasmid mit $T_3$ und $T_7$ Promotor-Gen, Ampicillin-Resistenz-Gen, fl origin, ColE-1 origin, lacI Gen, lacZ Gen und einer Multiple Cloning Site), der zuvor ebenfalls mit EcoRI und HindIII verdaut wurde, ligiert. Der Ligationsansatz wurde in Escherichia coli transformiert. Die erhaltenen Klone, selektiert auf Ampicillin, wurden durch Restriktion mit den Enzymen EcoRI und HindIII analysiert. Der resultierende Klon, pBS+ETI, enthält ein zusätzliches EcoRI/HindIII-Fragment mit einer Größe von ca. 539 bp (SEQ ID NO:9).

**b) Expressionsvektor**

Das Plasmid pBS+ETI wurde mit den Restriktionsenzymen EcoRI und HindIII restringiert, und das 539 bp große Fragment wurde isoliert. Der Expressionsvektor pBTacl (Boehringer Mannheim GmbH, Katalog Nr. 1081365 *(3)*, auf Basis von pUC8, H. Haymerle et al. (1986) *(21)*) wurde ebenfalls mit den Enzymen EcoRI und HindIII verdaut und das etwa 4.6 kb große Vektorfragment isoliert. Beide Fragmente wurden ligiert und zusammen mit dem Helferplasmid pUBS520 (Brinkmann et al. (1989) *(4)*), das das lac-Repressorgen enthält, in E. coli (DSM 5443) transformiert. Die Selektion der Klone erfolgte über die durch die Plasmide vermittelte Ampicillin- bzw. Kanamycin-Resistenz. Das erhaltene Plasmid pBTETI enthält, im Vergleich zum Ausgangsvektor pBTacl, ein zusätzliches EcoRI/HindIII-Fragment mit einer Größe von 539 bp und kann zur Expression von rekombinantem, nicht modifiziertem ETI verwendet werden (recETI).

In analoger Weise kann anstelle von DSM 5443 auch DSM 3689, der bereits ein I<sup>q</sup> Plasmid enthält, verwendet

werden. In diesem Fall ist das Helferplasmid pUBS520 nicht erforderlich.

Zum Zwecke der N-terminalen Mutation und Einführung eines neuen Promoters wurde eine Fusions-PCR durchgeführt. Mit den Oligonukleotiden ETI-1 und ETI-2 wurde der Promoter aus dem Plasmid pDS56/RBII (kommerziell erhältlich von der Firma Qiagen unter dem Namen pQE-6) amplifiziert (Amplifikationsprodukt A). Unter Verwendung der PCR-Primer ETI-3 und ETI-4 wurde die synthetische Sequenz, welche für ein ETI-Polypeptid kodiert, aus dem Plasmid pBTETI isoliert, wobei der Primer ETI-3 so konzipiert wurde, daß das amplifizierte Produkt für ein ETI-Polypeptid mit dem gewünschten Aminosäureaustausch (Ser) kodiert (Amplifikationsprodukt B). Die beiden Amplifikationsprodukte wurden via PCR und mit Hilfe der Primer ETI-1 und ETI-4 fusioniert. Das Fusionsprodukt wurde mit den beiden Restriktionsenzymen BamHI und HindIII gespalten und aufgereinigt. Das Plasmid pA27fd, EP-A 0 382 174 (USP 5,223,256) (12) wurde mit den beiden Restriktionsenzymen BamHI und HindIII (partiell) behandelt und das etwa 4600 bp große Vektorfragment isoliert. Vektorfragment und Fusionsfragment wurden ligiert und zusammen mit dem Helferplasmid pUBS520 (Brinkmann et al. 1989 (4)) in E.coli C600+ (DSM 5443) transformiert. Die Selektion der Klone erfolgte über die durch die Plasmide vermittelte Ampicillin- und Kanamycin-Resistenz. Das erhaltene Plasmid pETI-T2Lvs enthält, im Gegensatz zu dem Ausgangsplasmid pBTETI, ein zusätzliches HindIII-Fragment von etwa 350 bp.

## Tabelle 1: <u>PCR-Primer</u>

| Primer | Sequenz 5' -> 3' | |
|--------|------------------|---|
| ETI-1 | AAAGGATCCCTCGAGAAATCATAAAAA | (SEQ ID NO:5) |
| ETI-2 | CATAAGAATTCTGTTTCCTCTTTAATGAATTCTG | (SEQ ID NO:6) |
| ETI-3 | CAGAATTCTTATGTCATTATTAGA | (SEQ ID NO:7) |
| ETI-4 | AGAAGCTTTTATCAGCTG | (SEQ ID NO:8) |

**c) Expression von rekombinantem ETI-Polypeptid (SerETI) in E. coli**

Zur Überprüfung der Expressionsleistung wurde der E. coli Stamm DSM 5443 mit den Plasmiden pETI-T2Lvs und pUBS520 in LB-Medium (Sambrook et al.(1989) (38)) in Gegenwart von Ampicillin und Kanamycin (jeweils 50 μg/ml Endkonzentration) bis zu einer optischen Dichte (OD) von 0,6 bei 550 nm angezüchtet. Die Expression wurde durch Zugabe von 5 mM IPTG initiiert. Die Kultur wurde für weitere 4 Stunden inkubiert. Im Anschluß daran wurden die E. coli durch Zentrifugation gesammelt und in Puffer resuspendiert (50 mM Tris-HCl pH 8, 50 mM EDTA); durch Beschallung wurde die Lyse der E. coli herbeigeführt. Durch erneute Zentrifugation wurden die unlöslichen Proteinfraktionen (Inclusion Bodies) gesammelt und in oben genanntem Puffer durch Beschallung resuspendiert. Die Suspension wurde mit 1/4 Volumen Auftragspuffer (250 mM Tris-HCl pH 6.8, 0.01 M EDTA, 5% SDS, 5% Mercaptoethanol, 50% Glycerin und 0.005% Bromphenolblau) versetzt und mit Hilfe eines 12.5% SDS-Polyacrylamidgel analysiert. Als Kontrolle wurde die gleiche Präparation mit einer Kultur von E. coli (pETI-T2Lvs/pUBS520), die nicht mit IPTG versetzt worden war, durchgeführt und im Polyacrylamidgel aufgetragen. In der Präparation der IPTG-induzierten Kultur kann man, nach Anfärben des Gels mit 0.2% Coomassie-Blue R250 (gelöst in 30% Methanol und 10% Essigsäure) und Entfärben des Gels in einem Methanol-Essigsäure-Gemisch, eine deutliche Bande mit einem Molekulargewicht von etwa 22 kD erkennen. Diese Bande ist in der Präparation der nicht-induzierten E. coli Zellen nicht vorzufinden.

**Beispiel 2**

**Renaturierung und Reinigung von SerETI**

50 g Inclusion Bodies (IBs) wurden mit 0,1 M Tris/HCl, pH 8,5, 6 M Guanidin, 0,1 M DTE, 1 mM EDTA solubilisiert (90 min bei 25°C) und nach Einstellen des pH-Wertes auf 2,5 (HCl) gegen 3 mol/l Guanidin/HCl dialysiert. Das Dialysat wurde zentrifugiert (SS34, 13.000 Upm) und durch Konzentrieren über YM 10 auf $C_{Prot.}$ = 36,9 mg/ml eingestellt. Ein 1 l Reaktionsgefäß wurde mit 0,1 M Tris/HCl, pH 8,5, 1 mM EDTA, 1 mM GSH, 0,1 mM GSSG gefüllt. Die Renaturierung wurde bei 20°C durch 16-fache Zugabe des Dialysats im Zeitabstand von 30 min durchgeführt.

**Reinigung von SerETI**

**a) über Anionenaustauscher**

SerETI wird in 0,1 M Tris/HCl, pH 8,5, 1 mM EDTA, 1 mM GSH, 0,1 mM GSSG renaturiert. Das Renaturat wird mit $H_2O$ 1:2 verdünnt, mit HCl auf pH 8,0 eingestellt und auf eine mit 50 mM Tris/HCl, pH 8,0 äquilibrierte Q-Sepharose®-Säule aufgetragen (5 mg Protein/ml Gel). Nach Waschen der Säule mit dem Äquilibrierungspuffer und mit 50 mM $Na_2HPO_4/H_3PO_4$, pH 8,0 (jeweils fünf Säulenvolumen) erfolgt die Elution mit 50 mM $Na_2HPO_4/H_3PO_4$, pH 8,0, 0,2 M NaCl.

**b) über Kationenaustauscher**

Renaturiertes Ser-ETI wurde durch Zugabe von HCl auf pH 4,0 eingestellt und gegen 50 mM NaOAc/HCl, pH 4,0 dialysiert (Cross Flow). Das Dialysat wurde zentrifugiert (13.000 Upm, 30 min, SS 34) und auf eine mit 50 mM NaOAc/HCl, pH 4,0 äquilibrierte TSK-SP-Säule (Kationenaustauscher mit Sulfopropylseitengruppen, Merck, Darmstadt, Deutschland, Volumen 15 ml) aufgetragen. Nach Waschen der Säule mit dem Äquilibrierungspuffer und mit 50 mM NaOAc/HCl, pH 4,0, 0,1 M NaCl erfolgt die Elution 50 mM NaOAc/HCl, pH 4,0, 0,2 M NaCl.

Die Reinheit des Eluats wurde durch SDS-PAGE und über RP-HPLC überprüft.

Ergebnis:

Ser-ETI bindet unter den verwendeten Bedingungen an die TSK-SP-Säule und kann mit 0,2 M NaCl eluiert werden. SDS-PAGE- und RP-HPLC-Analytik ergeben eine Reinheit von > 95%.

**Beispiel 3**

**Vergleich der spezifischen Aktivität von SerETI, recETI und von ETI aus dem Samen von Erythrina caffra**

SerETI, recETI und ETI, isoliert aus dem Samen von E. caffra [ETI (Samen)], wurden gegen 50 mM $Na_2HPO_4/$ $H_3PO_4$, pH 8,0, 0.25 M NaCl dialysiert und auf eine Proteinkonzentration von 1.0 mg/ml eingestellt. Die Proteinkonzentration wurde durch Messung der UV-Absorption bei 280 nm bestimmt ($\varepsilon$ = 1,46 $cm^2/mg$).

**Bestimmung der ETI-Aktivität**

Die Hemmung von Trypsin durch ETI wird mit N-$\alpha$-Benzoyl-L-arginin-4-nitroanilid (BAPA) als Substrat gemessen. 40 µl einer Trypsin-Lösung (0,13 mg/ml 2 mM HCl) werden in einer Quarzküvette mit 60 µl Testpuffer (0,1 M Tris/HCl, pH 8,0) und 100 µl ETI-Lösung gemischt und 5 min bei 30°C inkubiert. Nach Zugabe von 800 µl BAPA-Lösung (10 mg BAPA x HCl/10 ml Testpuffer) wird der Extinktionsanstieg/min bei 405 nm bestimmt.

Die ETI-Aktivität wird nach folgender Formel ermittelt:

$$U/ml = [1 - E_{Probe}/E_{Trypsin}] \cdot C_{Trypsin} \cdot 0.328 \cdot V$$

$E_{Probe}$: Extinktionsanstieg/min der gehemmten Probe
$E_{Trypsin}$: Extinktionsanstieg/min des ungehemmten Trypsins
$C_{Trypsin}$: Trypsin-Konzentration im Testansatz
V: Vorverdünnung der ETI-Lösung

| Protein | Spez. Aktivität U/mg) |
|---|---|
| ETI (Samen) | 0.88 |

(fortgesetzt)

| Protein | Spez. Aktivität U/mg) |
|---------|----------------------|
| SerETI | 1,5 |
| rec.ETI | 1,07 |

Ergebnis: Die spezifische Aktivität von SerETI ist um 50% höher als die spezifische Aktivität des nach klassischen Verfahren aus dem Samen von E. caffra isolierten ETI.

**Beispiel 4**

**Kopplung von ETI an CNBr-Sepharose®**

170 mg gereinigtes SerETI bzw. ETI(Samen) oder rekombinantes ETI (hergestellt analog Beispiel 1 und 2) wurden gegen 0,05 M $H_3BO_3$/NaOH, pH 8,0, 0,5 M NaCl (Kupplungspuffer) dialysiert und mit 7,5 g CNBr-Sepharose® (über Nacht in 500 ml 1 mM HCl gequollen, danach abgesaugt und in Kupplungspuffer suspendiert) gemischt. Die Suspension wurde 90 min bei Raumtemperatur inkubiert, abgesaugt und über Nacht mit 400 ml 0,1 M Tris/HCl, pH 8,0 geschüttelt. Die SerETI-Sepharose® wurde abgesaugt und mit 0,7 M Arginin/$H_3PO_4$, pH 7,5 äquilibriert.

**Beispiel 5**

**Reinigung eines rekombinanten Plasminogenaktivators rPA**

rPA: rekombinantes tPA-Derivat, bestehend aus den Domänen Kringel 2 und Protease (Herstellung nach EP-A 0 382 174 (US Patent 5,223,256) (12))

54 mg rekombinanter Plasminogenaktivator rPA (Proteinkonzentration bestimmt über die Absorption bei 280 nm, Extinktionskoeffizient 1,69 cm$^2$/mg) wurden auf eine mit 0,7 M Arginin/$H_3PO_4$, pH 7,5 äquilibrierte ETI-Sepharose gegeben. Nach Waschen mit Äquilibrierungspuffer und mit 0,3 M Arginin/$H_3PO_4$, pH 7,0 (jeweils fünf Säulenvolumen) erfolgte die Elution mit 0,3 M Arginin/$H_3PO_4$, pH 4,5. Der Plasminogenaktivator-Gehalt im Eluat wurde mit S 2288 als Substrat bestimmt, vgl. Beispiel 6.

**Beispiel 6**

**Vergleich der Bindungskapazität von SerETI- und ETI(Samen)-Sepharose für r-PA**

ETI(Samen) und Ser-ETI wurden, entsprechend den Empfehlungen des Sepharose-Herstellers (Pharmacia, Freiburg) an CNBr-Sepharose ff gekuppelt. Die fertigen Säulen wurden mit 0.7 mol/l Arg/$H_3PO_4$, pH 7.5 äquilibriert, mit 2 MU r-PA/ml Gel beladen und nach Waschen mit 5 SV 0.7 mol/l Arg/$H_3PO_4$, pH 7.5, 0.5 M NaCl und mit 5 SV 0.3 mol/l Arg/$H_3PO_4$, pH 7.0 mit 0,3 mol/l Arg/$H_3PO_4$, pH 4.5 eluiert. Die Bindungskapazität wurde als eluiertes rPA (MU/ml Gel) bestimmt. Jedes Gel wurde fünfmal beladen und eluiert. Zwischen den einzelnen Schritten wurden die Gele unter Standardbedingungen regeneriert.

| Lauf | Bindungskapazität MU/ml Samen-ETI-Seph. | Ser-ETI-Seph. |
|------|------------------------------------------|---------------|
| 1 | 1.04 | 1.28 |
| 2 | 0.95 | 1.35 |
| 3 | 1.08 | 1.53 |
| 4 | 1.01 | 1.48 |
| 5 | 0.98 | 1.49 |

Die in der Tabelle zusammengefaßten Daten zeigen, daß die durch Kupplung von Ser-ETI an CNBr-Sepharose hergestellte Ser-ETI-Sepharose eine um den Faktor 1.5-fach höhere Bindungskapazität für r-PA besitzt als die durch Kupplung von Samen-ETI an CNBr-Sepharose entstandene Samen-ETI-Sepharose.

Bestimmung der Aktivität: (Kohnert et al.(1992) *(29)*):

200 µl Puffer (0,1 mol/l Tris HCl pH 7.5, 0.15 % Tween®80) und 200 µl rPA-Lösung, verdünnt mit Puffer zu einer Konzentration von 1 - 12 µg/ml, werden für 5 Minuten bei 37°C inkubiert. Der Test wird gestartet durch die Zugabe von 200 µl S 2288 (6 mmol/l (H-D-Ile-Pro-Arg-P-Nitroanilid-Dehydrochlorid, Kabi Vitrum, Schweden)), das ebenfalls auf 37°C temperiert war. Die amidolytische Aktivität wird berechnet aus der Zunahme der Absorption bei 405 nm innerhalb der ersten 2,5 Minuten, bei einem Extinktionskoeffizient für p-Nitroanilin von 9750 l/mol/cm.

**Referenzliste**

1) Aman et al.; Gene 40 (1985) 183

2) Beattie und Fowler; Nature 352 (1991) 548 - 549

3) Boehringer Mannheim GmbH, Katalog Nr. 1081365

4) Brinkmann et al.; Gene 85 (1989) 109 - 114

5) Brosius et al.; J. Mol. Biol. 148 (1981) 107 - 127

6) Chang et al.; Nature 198 (1977) 1056

7) Dalborge, H. et al.; FEBS Letters 266 (1990) 1 - 3

8) Davies et al.; Ann. Rev. Microbiol. 32 (1978) 469

9) DE-A 44 24 171

10) EP-A 0 219 874

11) EP-A 0 267 851

12) EP-A 0 382 174 (US-Patent 5,223,256)

13) EP-B 0 093 619

14) EP-B 0 112 122

15) EP-B 0 218 479

16) EP-B 0 241 022

17) Ghrayeb et al.; EMBO J. 3 (1984) 2437

18) Goeddel et al.; Nuc. Acids Res. 8 (1980) 4057

19) Hames, B.D.; Higgins, S.G.; Nucleic Acid Hybridization - A practical approach (1985) IRL Press, Oxford, England

20) Harris, T.J.R.; Protein Engineering 1 (1987) 449 - 458

21) Haymerle, H. et al.; Nucl. Acid Res. 14 (1986) 8615 - 8624

22) Heussen, C.; J. Biol. Chem. 259 (1984) 11635 - 11638

23) Heussen,C.; Haemostasis 11 (1982) P47 (Supplement)

24) Höltke und Kessler; "The DIG system user's guide for filter hybridization" (1990), Boehringer Mannheim GmbH, Germany.

25) Joubert, F.J.; Thrombosis and Haemostasis 57 (3) (1987) 356 - 360

26) Joubert, F.J.; Int. J. Biochem. 14 (1982) 187 - 193

27) Joubert, F.J.; Phytochemistry 21 (1982) 1213 - 1217

28) Kingsman, S.M. et al.; Tibtech 5 (1987) 53 - 57

29) Kohnert, U. et al.; Protein Engineering 5 (1992) 93 - 100

30) Lehle, K. et al.; J. Mol. Biol. 239 (1994) 276 - 284

31) Miller et al.; Bio/Technology 7 (1989) 698)

32) Nagai et al.; Nature 309 (1984) 810

33) Oka et al.; Proc. Natl. Acad. Sci. USA 82 (1985) 7212

34) Onesti, S. et al.; J. Mol. Recogn. 5 (1992) 105 - 114

35) Palva et al.; Proc. Natl. Acad. Sci. USA 79 (1982) 5582

36) Porath, J. & Olin, B.; Biochemistry 22 (1983), 1621 - 1630

37) Powell et al.; Appl. Environ. Microbiol. 54 (1988) 655

38) Sambrook et al.; Molecular Cloning: A laboratory manual (1989) Cold Spring Harbor Laboratory Press,New York, USA

39) Sambrook et al., "Expression of cloned genes in E. coli" in Molecular Cloning: A laboratory manual (1989) Cold Spring Harbor Laboratory Press,New York, USA

40) Sambrook, J. et al; Section IX, "A hybridization of radiolabelled probes to immobilized nucleic acid", in Molecular Cloning: A laboratory manual (1989), Cold Spring Harbor Laboratory Press, New York, USA, 947 - 962

41) Shimatake et al.; Nature 292 (1981) 128

42) Shine et al.; Nature (1975) 25434;

43) Stratagene, US, Catalogue No. 211201

44) Studier et al.; J. Mol. Biol. 189 (1986) 113

45) Teixeira et al.; Biochimica et Biophysica Acta 1217 (1994) 16-22

46) Teixeira et al.; Biochemica et Biophysica Acta 1217 (1994) 23 - 28

47) US-Patent 4,336,336

48) US-Patent 4,551,433

49) US-Patent 4,689,406

50) US-Patent 4,747,056

51) US-Patent 4,902,623

52) US-Patent 4,933,434

53) US-Patent 5,223,256

54) WO 90/09437

55) WO 91/11520

**Patentansprüche**

1. Verfahren zur Reinigung einer Serinprotease aus einem Proteingemisch durch Bindung der Serinprotease an ein immobilisiertes Polypeptid mit der Aktivität eines Inhibitors DE-3 aus Erythrina caffra, Entfernen der ungebundenen Anteile aus dem Proteingemisch, Ablösen der Serinprotease vom Inhibitor, Trennung des immobilisierten Inhibitors von der löslichen Serinprotease und Isolierung der Serinprotease, **dadurch gekennzeichnet, daß** als Polypeptid ein Polypeptid verwendet wird, welches eine Aminosäuresequenz besitzt, die funktionell analog zur SEQ ID NO: 2 ist, zwei Disulfidbrücken besitzt, in einem Teilbereich zu mehr als 85% homolog zum Bereich 39 - 139 dieser Aminosäuresequenz ist, N-terminal mit SEQ ID NO:4 oder mit einer um Methionin N-terminal verlängerten SEQ ID NO:4 beginnt und eine spezifische Aktivität von 1,07 U/mg oder höher gegen Trypsin und eine Bindekapazität von 1,25 MU/ml oder mehr für Plasminogenaktivatoren besitzt.

**2.** Verfahren nach Anspruch 1, **dadurch gekennzeichnet, daß** ein Polypeptid, welches N-terminal mit SEQ ID NO: 4 beginnt, verwendet wird.

**3.** Verfahren nach Anspruch 1 oder 2, **dadurch gekennzeichnet, daß** das Polypeptid codiert wird

a) von einer Nukleinsäure gemäß SEQ ID NO:1,

b) von einer Nukleinsäure, die mit einer Sequenz, die komplementär zu der in SEQ ID NO:1 gezeigten DNA-Sequenz ist, unter stringenten Bedingungen hybridisiert und N-terminal mit einer Nukleinsäuresequenz beginnt, welche für SEQ ID NO:4 codiert,

c) von einer Nukleinsäure, die ohne die Degeneration des genetischen Codes mit einer Sequenz, die komplementär zu der in a) oder b) genannten Sequenz ist, unter stringenten Bedingungen hybridisieren würde.

**4.** Verfahren nach den Ansprüchen 1 - 3, **dadurch gekennzeichnet, daß** die Serinprotease ein Plasminogenaktivator ist.

**5.** Verfahren zur Herstellung eines Polypeptids, welches die Aktivität eines Inhibitors DE-3 aus Erythrina caffra besitzt und reversibel und selektiv Serinproteasen aus einem Proteingemisch bindet, durch Kultivieren von prokaryontischen oder eukaryontischen Wirtszellen, die mit einer Nukleinsäure, die für das genannte Polypeptid codiert, in einer Weise transformiert oder transfiziert sind, die es den Wirtszellen erlauben, unter geeigneten Nährstoffbedingungen das genannte Polypeptid zu exprimieren und Isolierung des genannten Polypeptids, **dadurch gekennzeichnet, daß** das Polypeptid eine Aminosäuresequenz besitzt, welche funktionell analog zur SEQ ID NO:2 ist, in einem Teilbereich zu mehr als 85 % homolog zum Bereich der Aminosäuren 39 - 139 zu dieser Sequenz ist, zwei Disulfidbrücken besitzt, N-terminal mit SEQ ID NO:4 oder mit einer um Methionin N-terminal verlängerten SEQ ID NO:4 beginnt und eine spezifische Aktivität von 1,07 U/mg oder höher gegen Trypsin und eine Bindekapazität von 1,25 MU/ml oder mehr für Plasminogenaktivatoren besitzt.

**6.** Verfahren nach Anspruch 5, **dadurch gekennzeichnet, daß** das Polypeptid N-terminal mit SEQ ID NO:4 beginnt.

**7.** Verfahren nach Anspruch 5 oder 6, **dadurch gekennzeichnet, daß** das Polypeptid codiert wird

a) von einer Nukleinsäure gemäß SEQ ID NO:1,

b) von einer Nukleinsäure, die mit einer Sequenz, die komplementär zu der in SEQ ID NO:1 gezeigten DNA-Sequenz ist, unter stringenten Bedingungen hybridisiert und N-terminal mit einer Nukleinsäuresequenz beginnt, welche für SEQ ID NO:4 codiert,

c) von einer Nukleinsäure, die ohne die Degeneration des genetischen Codes mit einer Sequenz, die komplementär zu einer der in a) oder b) genannten Sequenzen ist, unter stringenten Bedingungen hybridisieren würde.

**8.** Isolierte Nukleinsäure gemäß SEQ ID NO:1, die für ein Polypeptid mit der Aktivität eines Inhibitors DE-3 aus Erythrina caffra codiert.

**9.** Polypeptid, welches die Aktivität eines Inhibitors DE-3 aus Erythrina caffra besitzt und reversibel und selektiv Serinproteasen aus einem Proteingemisch bindet, erhältlich durch Kultivieren von prokaryontischen oder eukaryontischen Wirtszellen, die mit einer Nukleinsäure, die für das genannte Polypeptid codiert, in einer Weise transformiert oder transfiziert sind, die es den Wirtszellen erlauben, unter geeigneten Nährstoffbedingungen das genannte Polypeptid zu exprimieren und Isolierung des genannten Polypeptids, **dadurch gekennzeichnet, daß** das Polypeptid eine Aminosäuresequenz besitzt, welche funktionell analog zur SEQ ID NO:2 ist, in einem Teilbereich zu mehr als 85% homolog zum Bereich der Aminosäuren 39 - 139 dieser Sequenz ist, zwei Disulfidbrücken besitzt, N-terminal mit SEQ ID NO:4 oder mit einer um Methionin N-terminal verlängerten SEQ ID NO:4 beginnt und eine spezifische Aktivität von 1,07 U/mg oder höher gegen Trypsin und eine Bindekapazität von 1,25 MU/ml oder mehr für Plasminogenaktivatoren besitzt.

**10.** Polypeptid nach Anspruch 9, **dadurch gekennzeichnet, daß** es N-terminal mit SEQ ID NO:4 beginnt.

**11.** Polypeptid nach Anspruch 9 oder 10, **dadurch gekennzeichnet, daß** es eine Bindekapazität für Gewebsplasminogenaktivatoren von 1,25 MU/ml und mehr besitzt.

**Claims**

**1.** Method for purifying a serine protease from a protein mixture by binding the serine protease to an immobilized polypeptide having the activity of an inhibitor DE-3 from Erythrina caffra, removing the unbound components from the protein mixture, detaching the serine protease from the inhibitor, separating the immobilized inhibitor from the soluble serine protease and isolating the serine protease, **characterized in that** a polypeptide is used as the polypeptide which has an amino acid sequence which is functionally analogous to SEQ ID NO:2, has two disulfide bridges, is more than 85 % homologous to region 39 - 139 of this amino acid sequence in one section, begins N-terminally with SEQ ID NO:4 or with SEQ ID NO:4 extended N-terminally by methionine and has a specific activity of 1.07 U/mg or more towards trypsin and a binding capacity of 1.25 MU/ml or more for plasminogen activators.

**2.** Method as claimed in claim 1, **characterized in that** a polypeptide is used which begins N-terminally with SEQ ID NO:4.

**3.** Method as claimed in claim 1 or 2, **characterized in that** the polypeptide is coded

a) by a nucleic acid according to SEQ ID NO:1,

b) by a nucleic acid that hybridizes under stringent conditions with a sequence that is complementary to the DNA sequence shown in SEQ ID NO:1 and begins N-terminally with a nucleic acid sequence which codes for SEQ ID NO:4,

c) by a nucleic acid which, without the degeneracy of the genetic code, would hybridize under stringent conditions with a sequence which is complementary to the sequence mentioned in a) or b).

**4.** Method as claimed in claims 1 - 3, **characterized in that** the serine protease is a plasminogen activator.

**5.** Process for producing a polypeptide which has the activity of an inhibitor DE-3 from Erythrina caffra and reversibly and selectively binds serine proteases from a protein mixture by culturing prokaryotic or eukaryotic host cells that have been transformed or transfected with a nucleic acid which codes for the said polypeptide that enables the host cells to express the said polypeptide under suitable nutrient conditions and isolating the said polypeptide, **characterized in that** the polypeptide has an amino acid sequence which is functionally analogous to SEQ ID NO:2, is more than 85 % homologous to the region of amino acids 39 - 139 of this sequence in one section has two disulfide bridges, begins N-terminally with SEQ ID NO:4 or with SEQ ID NO:4 extended N-terminally by methionine and has a specific activity of 1.07 U/mg or more towards trypsin and a binding capacity of 1.25 MU/ml or more for plasminogen activators.

**6.** Process as claimed in claim 5, **characterized in that** the polypeptide begins N-terminally with SEQ ID NO:4.

**7.** Process as claimed in claim 5 or 6, **characterized in that** the polypeptide is coded

a) by a nucleic acid according to SEQ ID NO:1,

b) by a nucleic acid that hybridizes under stringent conditions with a sequence that is complementary to the DNA sequence shown in SEQ ID NO:1 and begins N-terminally with a nucleic acid sequence which codes for SEQ ID NO:4,

c) by a nucleic acid which, without the degeneracy of the genetic code, would hybridize under stringent conditions with a sequence which is complementary to the sequence mentioned in a) or b).

**8.** Isolated nucleic acid having SEQ ID NO:1 which codes for a polypeptide having the activity of an inhibitor DE-3 from Erythrina caffra.

**9.** Polypeptide which has the activity of an inhibitor DE-3 from Erythrina caffra and reversibly and selectively binds serine proteases from a protein mixture obtainable by culturing prokaryotic or eukaryotic host cells that have been transformed or transfected with a nucleic acid which codes for the said polypeptide that enables the host cells to express the said polypeptide under suitable nutrient conditions and isolating the said polypeptide, **characterized in that** the polypeptide has an amino acid sequence which is functionally analogous to SEQ ID NO:2, is more than 85 % homologous to the region of amino acids 39 - 139 of this sequence in one section, has two disulfide bridges, begins N-terminally with SEQ ID NO:4 or with SEQ ID NO:4 extended N-terminally by methionine and has a specific activity of 1.07 U/mg or more towards trypsin and a binding capacity of 1.25 MU/ml or more for plasminogen activators.

**10.** Polypeptide as claimed in claim 9, **characterized in that** it begins N-terminally with SEQ ID NO:4.

**11.** Polypeptide as claimed in claim 9 or 10, **characterized in that** it has a binding capacity for tissue plasminogen activators of 1.25 MU/ml or more.

**Revendications**

**1.** Procédé de purification d'une protéase sérique à partir d'un mélange de protéines par liaison de la protéase sérique à un polypeptide immobilisé ayant l'activité d'un inhibiteur DE-3 de *Erythrina caffra*, élimination de la fraction non liée du mélange de protéines, détachement de la protéase sérique de l'inhibiteur, séparation de l'inhibiteur immobilisé de la protéase sérique soluble et isolement de la protéase sérique, **caractérisée en ce que** comme polypeptide on utilise un polypeptide qui dispose d'une séquence d'acides aminés qui est analogue sur le plan fonctionnel à la SEQ ID N°2, qui dispose de deux ponts disulfure, qui est, dans un domaine partiel, homologue à plus de 85 % au domaine 39-139 de cette séquence d'acides aminés, qui commence à l'extrémité N-terminale par SEQ ID N°4 ou par SEQ ID N°4 prolongée à l'extrémité N-terminale par une méthionine et qui dispose d'une activité spécifique de 1,07 U/mg ou plus contre la trypsine et d'une capacité de liaison de 1,25 MU/ml ou plus pour des activateurs du plasminogène.

**2.** Procédé selon la revendication 1, **caractérisé en ce qu'**un polypeptide dont l'extrémité N-terminale commence avec SEQ ID N°4 est utilisé.

**3.** Procédé selon la revendication 1 ou 2, **caractérisé en ce que** le polypeptide est codé

a) par un acide nucléique selon SEQ ID N°1,

b) par un acide nucléique qui s'hybride avec une séquence complémentaire à la séquence d'ADN présentée dans SEQ ID N°1 dans des conditions stringentes et qui commence à son extrémité N-terminale par une séquence d'acides nucléiques qui code SEQ ID N°4,

c) par un acide nucléique qui, sans dégénérescence du code génétique, s'hybriderait, dans des conditions stringentes, avec une séquence qui est complémentaire à la séquence citée en a) ou b).

**4.** Procédé selon les revendications 1 à 3, **caractérisé en ce que** la protéase sérique est un activateur du plasminogène.

**5.** Procédé de fabrication d'un polypeptide qui dispose de l'activité d'un inhibiteur DE-3 de *Erythrina caffra* et qui se lie de manière réversible et sélective à des protéases sériques d'un mélange de protéines, par culture de cellules hôtes procaryotes ou eucaryotes qui sont transformées ou transfectées avec un acide nucléique qui code le polypeptide cité, de telle sorte que les cellules hôtes puissent, dans des conditions nutritionnelles adaptées, exprimer le polypeptide cité, et par isolement du polypeptide cité, **caractérisé en ce que** le polypeptide contient une séquence d'acides aminés qui est analogue sur le plan fonctionnel à SEQ ID N°2, qui est, dans un domaine partiel, homologue à plus de 85 % au domaine des acides aminés 39-139 de cette séquence d'acides aminés, qui dispose de deux ponts disulfure, qui commence à l'extrémité N-terminale par SEQ ID N°4 ou par SEQ ID N°4 prolongée à l'extrémité N-terminale par une méthionine et qui dispose d'une activité spécifique de 1,07 U/mg ou plus contre la trypsine et d'une capacité de liaison de 1,25 MU/ml ou plus pour des activateurs du plasminogène.

**6.** Procédé selon la revendication 5, **caractérisé en ce que** le polypeptide commence, à son extrémité N-terminale, par SEQ ID N°4.

**7.** Procédé selon la revendication 5 ou 6, **caractérisé en ce que** le polypeptide est codé

a) par un acide nucléique selon SEQ ID N°1,

b) par un acide nucléique qui s'hybride avec une séquence complémentaire à la séquence d'ADN présentée dans SEQ ID N°1 dans des conditions stringentes et qui commence à son extrémité N-terminale par une séquence d'acides nucléiques qui code SEQ ID N°4,

c) par un acide nucléique qui, sans dégénérescence du code génétique, s'hybriderait, dans des conditions stringentes, avec une séquence qui est complémentaire aux séquences citées en a) ou b).

**8.** Acide nucléique isolé selon SEQ ID N°1, qui code un polypeptide ayant l'activité d'un inhibiteur DE-3 de *Erythrina caffra*.

**9.** Polypeptide qui dispose de l'activité d'un inhibiteur DE-3 de *Erythrina caffra* et qui se lie de manière réversible et sélective aux protéases sériques d'un mélange de protéines, obtenu par culture de cellules hôtes procaryotes et eucaryotes, qui sont transformées ou transfectées avec un acide nucléique qui code le polypeptide cité, de telle sorte que les cellules hôtes puissent, dans des conditions nutritionnelles adaptées, exprimer le polypeptide cité, et par isolement du polypeptide cité, **caractérisé en ce que** le polypeptide dispose d'une séquence d'acides aminés qui est analogue sur le plan fonctionnel à SEQ ID N°2, qui est, dans un domaine partiel, homologue à plus de 85 % au domaine des acides aminés 39-139 de cette séquence, qui dispose de deux ponts disulfure, qui commence à l'extrémité N-terminale par SEQ ID N°4 ou par SEQ ID N°4 prolongée à l'extrémité N-terminale par une méthionine et qui dispose d'une activité spécifique de 1,07 U/mg ou plus contre la trypsine et d'une capacité de liaison de 1,25 MU/ml ou plus pour des activateurs du plasminogène.

**10.** Polypeptide selon la revendication 9, **caractérisé en ce qu'**il commence, à son extrémité N-terminale, par SEQ ID N°4.

**11.** Polypeptide selon la revendication 9 ou 10, **caractérisé en ce qu'**il dispose d'une capacité de liaison à des activateurs du plasminogène tissulaire de 1,25 MU/ml et plus.